# EUROPÄISCHE PATENTSCHRIFT

(11) **EP 1 090 899 B1**
(45) Veröffentlichungstag und Bekanntmachung des Hinweises auf die Patenterteilung: **21.06.2006**
(21) Anmeldenummer: 00121800.7
(22) Anmeldetag: 05.10.2000
(51) Int. Cl.: C07C 45/50, C07C 45/72, C07C 29/141

(54) **Verfahren zur Herstellung von 1,3-Diolen**
Process for the preparation of 1,3-diols
Procédé de préparation de 1,3-dioles

(30) Priorität: 06.10.1999 DE 19948112
(43) Veröffentlichungstag der Anmeldung: 11.04.2001
(73) Patentinhaber: BASF Aktiengesellschaft, 67056 Ludwigshafen (DE)
(72) Erfinder: Kratz, Detlef, 69121 Heidelberg (DE); Rust, Harald, 67435 Neustadt (DE); Krokoszinski, Roland, 67273 Weisenheim (DE); Helf, Volker, 67304 Eisenberg (DE)
(74) Vertreter: Kinzebach, Werner

(56) Entgegenhaltungen:
- WO-A-93/10071
- WO-A-97/16401
- JP-A- 2 040 333
- US-A- 5 663 452
- MARGHERI, GABRIELE ET AL: "Oligomerization of aldehydes catalyzed by cobalt carbonyl complexes" JOURNAL OF MOLECULAR CATALYSIS A: CHEMICAL ( 1998 ), 132(2-3), 189-201 , XP002251383

## Beschreibung

Die Erfindung betrifft ein Verfahren zur Herstellung von 1,3-Diolen mit sechs oder mehr Kohlenstoffatomen mittels Aldolreaktion.

1,3-Diole mit sechs oder mehr Kohlenstoffatomen im Grundgerüst sind vielseitig verwendete Einsatzstoffe der chemischen Industrie. Sie sind beispielsweise bei der Herstellung von Polyestern, Polyurethanen, Lackrohstoffen, Dispersionen und Weichmachern brauchbar.

Zu derartigen Stoffen gehören 1,3-Diole mit acht Kohlenstoffatomen, beispiels-weise 2-Ethylhexandiol-1,3 (EHD), 2,2-Dimethylhexandiol-1,3 (DMHD), 2,2,4-Trimethylpentandiol-1,3 (TMPD) und 2-Ethyl-4-Methylpentandiol-1,3 (EMPD). Diese Stoffe finden zusätzlich auch als Insektenabwehrstoffe Verwendung.

Aus der WO A 97/16401 ist bekannt, derartige 1,3-Diole durch Hydroformylierung eines Olefins zu dem entsprechenden Aldehyd, anschliessende Aldolreaktion mit einem weiteren Aldehyd unter Zugabe eines Aldolisierungskatalysators und anschliessende Hydrierung herzustellen.

Die WO 95/07254 beschreibt ein Verfahren zur Gewinnung von EHD durch basenkatalysierte Umsetzung von n-Butyraldehyd (n-BA) in Gegenwart eines Phasentransferkatalysators. Die dort angegebene Ausbeute liegt bei etwa 56%.

Die Herstellung von EHD durch Aldoladdition wird auch in JP 2040-333-A beschrieben. Dabei wird n-BA unter der Einwirkung von NaOH oder KOH in Butanol zu einem Gemisch aus 2-Ethyl-3-hydroxyhexanal, dem sich daraus ableitenden Dehydratisierungsprodukt 2-Ethylhexenal und höher siedenden aldolartigen Kondensationsprodukten umgesetzt. Die Selektivität dieser Aldolreaktion liegt bei 86%, der erzielte Umsatz bei etwa 58%. Das so erhaltene Gemisch wird mit Essigsäure neutralisiert und anschliessend einer katalytischen Hydrierung an einem Raney-Nickel-Katalysator unterworfen.

In der JP-A 1299-240 wird ein ähnliches Verfahren beschrieben, bei dem Natriummethylat in Butanol als Katalysator dient.

Aus der US-A 4,225,726 ist die Verwendung von Zinn oder Zinnoxid als Katalysator zur Herstellung des Buttersäureesters von beispielsweise EHD bekannt.

Die vorstehend beschriebenen Verfahren weisen jedoch die folgenden Nachteile auf:

Der nicht vollständige Umsatz an n-BA bewirkt, dass bei der Hydrierung n-Butanol als nicht zu vernachlässigendes Nebenprodukt erhalten wird. n-BA könnte zwar vor der Hydrierung abgetrennt werden, dies ist jedoch mit weiterem zusätzlichen Aufwand verbunden.

Die bekannten Verfahren verwenden ausserdem zur Aldolisierung Katalysatoren. Wenn bei den nachfolgenden Schritten, etwa einer Hydrierung oder Destillation, der Aldolisierungskatalysator noch anwesend ist, kann das Aldoladditionsprodukt - im Falle der Verwendung von n-BA nämlich 2-Ethyl-3-hydroxyhexanal - wieder gespalten werden; des weiteren kann 2-Ethyl-3-hydroxyhexanal auch die Ausbeute mindernde Folgereaktionen eingehen. Damit ist in diesem Falle eine umständliche Abtrennung des Ka-talysators, beispielsweise durch Neutralisation, Waschen und Absorption, erforderlich, um Ausbeuteverluste zu vermeiden. Ebenso kann der Aldolisierungskatalysator aber auch die Effizienz der Hydrierung beeinträchtigen oder sogar den Hydrierkatalysator vergiften.

Die Bildung des erwähnten Dehydratisierungsproduktes kann in Gegenwart eines Katalysators nicht vermieden werden und bewirkt einen zusätzlichen Ausbeuteverlust.

Der Weg zur Herstellung des Diols über einen Ester, in der US-A 4,225,726 beispielsweise die Herstellung von EHD über den Buttersäureester, erfordert einen zusätzlichen Verfahrensschritt. Durch Verseifung des Esters entsteht zwar EHD, d.h. das gewünschte Diol, aber zusätzlich auch die Säurekomponente des Esters, wie Buttersäure oder ein Salz der Buttersäure, als Koppelprodukt. Die Entfernung derartiger Koppelprodukte ist wiederum mit einigem Aufwand verbunden.

Bei der Aldolisierungsreaktion entstehen weiterhin eine Reihe von Nebenprodukten wie Ester, Ether, Aldoxane, Halb- und Vollacetale des Aldehyds, die nach der Hydrierung zu einem breiten Nebenproduktspektrum führen. Dies wiederum erschwert die Gewinnung des reinen Diols durch destillative Aufarbeitung erheblich.

WO 93/10071 beschreibt die Herstellung von β-Hydroxyaldehyden oder -ketonen durch Aldolreaktion von z.B. Acetophenon mit Formaldehyd in Abwesenheit eines Katalysators. Aufgabe der Erfindung ist es daher, ein einfaches und kostengünstiges Verfahren zur Herstellung von 1,3-Diolen bereitzustellen, das insbesondere die Nachteile der vorstehend erwähnten üblichen Verfahren vermeidet oder zumindest verringert.

Es wurde nun gefunden, dass sich 1,3-Diole mit sechs oder mehr Kohlenstoffatomen in einfacher Weise, durch thermische Behandlung von Alkanalen herstellen lassen.

Erfindungsgemäss wird also die vorstehend erwähnte Aufgabe durch ein Verfahren zur Herstellung von 1,3-Diolen mit sechs oder mehr Kohlenstoffatomen gelöst, wobei das Verfahren die Schritte umfasst:
a) Bereitstellen mindestens eines Alkanals mit 3 oder mehr Kohlenstoffatomen,
b) thermische Behandlung des Alkanals ohne basischen Katalysator,
c) Hydrieren des in Stufe b) gebildeten Aldoladditionsprodukts zum 1,3-Diol, und
d) Gewinnung des 1,3-Diols.

Das erfindungsgemässe Verfahren geht gemäss Stufe a) aus von mindestens einem Alkanal mit drei oder mehr Kohlenstoffatomen z.B. 3 bis 10 Kohlenstoffatomen, bevorzugt 3 bis 5 ohlenstoffatomen und insbesondere 4 Kohlenstoffatomen. Insbesondere handelt es sich dabei um n-Butyraldehyd und i-Butyraldehyd. Die Bereitstellung des Alkanals kann durch übliche Verfahren zur Herstellung von Alkanalen erfolgen. Vorzugsweise erfolgt die Bereitstellung des Alkanals durch Oxo-Synthese, insbesondere ausgehend von Ethylen oder Propylen. Die Oxo-Synthese ist bekannt, siehe beispielsweise Ullmann's Encyclopedia of Industrial Chemistry, Vol. A18, 321, 5^{th} Edition. Bei der Oxo-Synthese wird das Olefin mit Kohlenmonoxid und Wasserstoff in Anwesenheit eines Übergangsmetallkatalysators - in der Regel Rhodium oder Cobalt - unter Druck und bei erhöhter Temperatur umgesetzt. Bei der Oxo-Synthese von Olefinen mit 3 oder mehr Kohlenstoffatomen erhält man im Allgemeinen ein Gemisch von Isomeren des betreffenden Alkanals. So wird beispielsweise bei der Oxo-Synthese ausgehend von Propylen ein Gemisch von i-Butyraldehyd (i-BA) und n-Butyraldehyd (n-BA) erhalten. Je nach Verfahrensbedingungen kann das Verhältnis zwischen i-BA und n-BA von etwa 50/50 bis 5/95 schwanken. Das erfindungsgemässe Verfahren kann ausgehend von einem Alkanal oder einem Gemisch von 2 oder mehreren Alkanalen bliebiger Zusammensetzung durchgeführt werden, wobei die Alkanale vorzugsweise 3 bis 10 Kohlenstoffatome aufweisen.

In Stufe b) wird das Alkanal einer thermischen Behandlung in Abwesenheit eines basischen Katalysators, wie er üblicherweise für eine Aldoladdition verwendet wird, unterworfen. Bei der thermischen Behandlung kommt es zu einer thermisch induzierten Aldoladdition unter Bildung des entsprechenden Aldoladditionsproduktes. Die thermische Behandlung erfolgt vorzugsweise im Rahmen einer Destillation oder Hydrierung des Alkanals. Das bei der Bereitstellung des Alkanals erhaltene Rohprodukt kann beispielsweise einer Destillation unterworfen werden, um das Alkanal zu reinigen oder ein Alkanalgemisch aufzutrennen. Bei der Destillation kommt es, wie erwähnt, zu einer thermisch induzierten Aldoladdition unter Bildung des entsprechenden Aldoladditionsproduktes. Dieses siedet höher als das entsprechende Alkanal und reichert sich daher in dem bei der Destillation anfallenden hochsiedenden Anteil an. Um das 1,3-Diol zu erhalten, muss der hochsiedende Anteil einer Hydrierung unterworfen werden. Diese erfolgt unter den für die Hydrierung von Alkanalen üblichen Bedingungen, also in Anwesenheit eines Hydrierkatalysators, wie beispielsweise in der DE 12 69 605 beschrieben, in der Flüssig- oder Gasphase. Als Katalysatoren in Betracht kommen beispielsweise Kupferchromit, geträgerte Nickel-, Kupfer- oder Cobaltkatalysatoren, die z.B. mit Mo, Mn oder Cr dotiert sein können, wobei als Trägermaterialien z.B. Kieselsäure, Kieselgur, Kohle, Zirkonoxid, Siliciumcarbid und dergleichen in Frage kommen. Weiter sind Edelmetalle wie Pd, Pt oder Rh, enthaltende Katalysatoren geeignet. Der Druck, bei dem die Hydrierung durchgeführt wird, liegt vorzugsweise im Bereich von 1 bis 250 bar, insbesondere 10 bis 150 bar. Die Temperatur liegt im Allgemeinen im Bereich von 50 bis 250°C, insbesondere 100 bis 210°C. Vorzugsweise wird der hochsiedende Anteil mit dem Aldoladditionsprodukt zusammen mit dem Alkanal hydriert.

Gemäss einer weiteren Ausführungsform erfolgt die thermische Behandlung im Rahmen einer Hydrierung eines Alkanals. Die Hydrierung erfolgt unter den oben angegebenen Bedingungen, wobei sich das Aldoladditionsprodukt bildet. Gleichzeitig wird dieses Aldoladditionsprodukt zum 1,3-Diol reduziert. Dabei erhält man bei Hydrierung eines reinen Alkanals das entsprechende 1,3-Diol, während man bei Hydrierung eines Alkanalgemisches ein Gemisch der entsprechenden 1,3-Diole erhält.

Gemäss Stufe c) wird dann das 1,3-Diol in üblicher Weise gewonnen. Vorzugsweise erfolgt dies durch Destillation des bei der Hydrierung anfallenden und das 1,3-Diol enthaltenden Produktes. Die leichtsiedenden Anteile, nämlich das bei der Hydrierung des Alkanals gebildete Alkanol wird abdestilliert, wobei sich das 1,3-Diol im hochsiedenden Anteil anreichert. Dieser wird einer weiteren Destillation unterzogen, bei welcher das 1,3-Diol abdestilliert wird und trotz der hohen Anzahl an Nebenkomponenten überraschenderweise in reiner Form erhalten werden kann. Die Destillation kann kontinuierlich oder diskontinuierlich erfolgen. Die Destillationsbedingungen richten sich nach dem betreffenden 1,3-Diol. Vorzugsweise verwendet man dazu eine Kolonne. Besonders geeignet sind Packungs- oder Füllkörperkolonnen, die sich gegenüber Kolonnen mit Glocken- oder Ventilböden durch geringere Differenzdrücke auszeichnen. Dadurch ist die thermische Belastung des zu destillierenden Produktes minimiert, sodass im Wesentlichen keine Zersetzungsreaktionen im Kolonnensumpf zu beobachten sind. Eine Zersetzungsreaktion kann zu leichtsiedenden Verunreinigungen im 1,3-Diol führen. Im Allgemeinen arbeitet man im Vakuum, beispielsweise bei einem Druck im Bereich von 1 bis 500 mbar, vorzugsweise 5 bis 250 mbar und insbesondere 10 bis 100 mbar. Die Destillationstemperatur richtet sich nach dem gewählten Druck und dem betreffenden 1,3-Diol, wobei die Sumpftemperatur maximal 200°C betragen sollte.

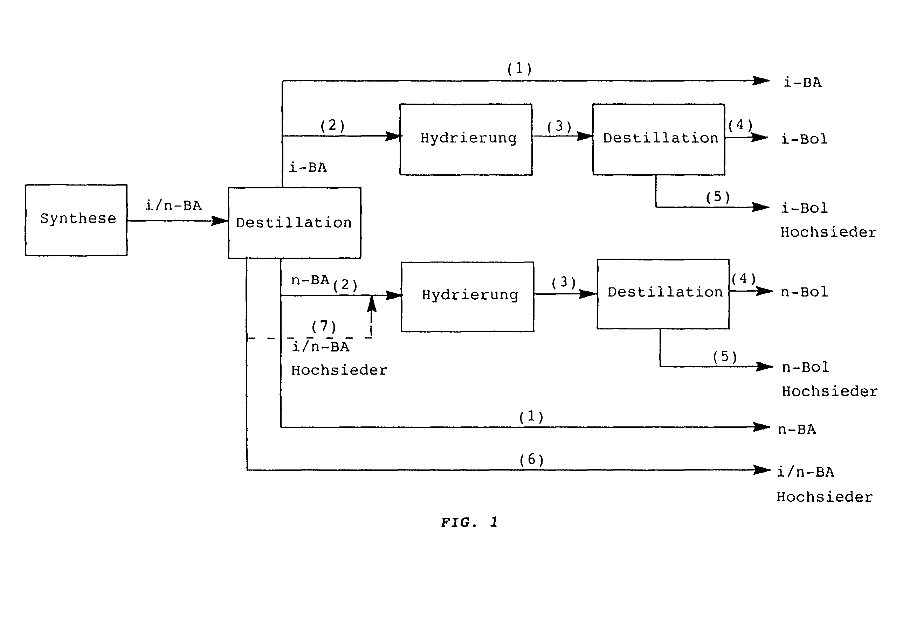

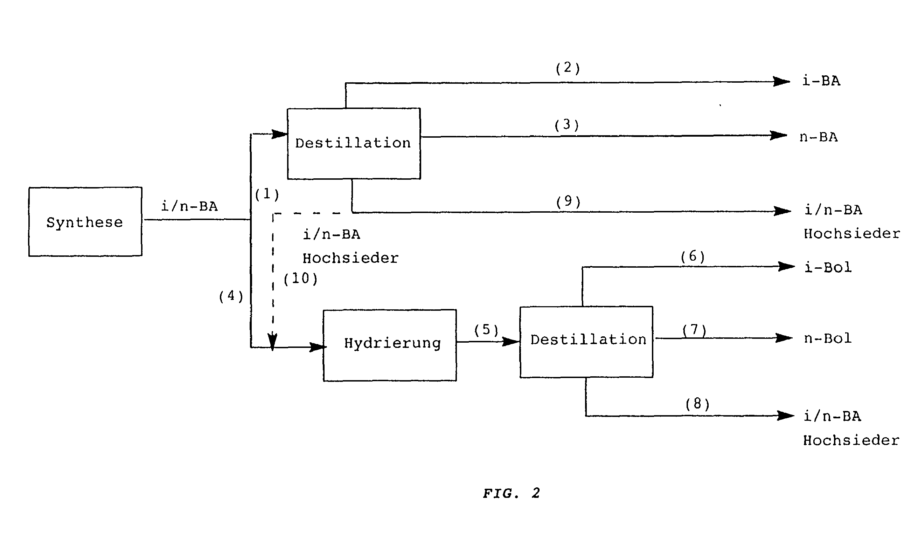

Nachfolgend wird das erfindungsgemässe Verfahren unter Bezug auf die Figuren 1 und 2 erläutert. Die Figuren 1 und 2 zeigen jeweils eine schematische Darstellung einer Ausführungsform des erfindungsgemässen Verfahrens. Dabei wird beispielhaft auf i-Butyraldehyd und n-Butyraldehyd als Alkanale Bezug genommen. Das als Ausgangsgemisch eingesetzte Rohgemisch aus i-Butyraldehyd und n-Butyraldehyd, wie es beispielsweise durch Oxo-Synthese aus Propylen erhalten wird, wird gemäss Figur 1 zuerst durch Destillation in die i- und n- Butyraldehyde aufgetrennt. Ein beliebiger Teil kann dabei als Reinaldehyd entnommen werden, siehe die Ströme (1). Der verbleibende Anteil an Reinaldehyden wird einer Hydrierung zu den entsprechenden Alkoholen unterworfen, siehe die Ströme (2). Die Hydrierung erfolgt unter den oben angegebenen Bedingungen. Bei der Hydrierung kommt es zur Bildung des Aldoladditionsproduktes, das gleichzeitig zu dem entsprechenden 1,3-Diol reduziert wird.

Das Produkt der Hydrierung wird dann einer Destillation zugeführt (siehe die Ströme (3)), um den gebildeten Alkohol, nämlich n-Butanol bzw. i-Butanol (n-Bol bzw. i-Bol) zu gewinnen. Dies wird durch die Ströme (4) veranschaulicht. Die Destillation erfolgt in üblicher Weise in einer oder mehreren Kolonnen, beispielsweise mit einer Packungskolonne. Druck und Temperatur richten sich nach dem betreffenden Alkohol.

Das 1,3-Diol reichert sich im hochsiedenden Anteil der Destillation an, bei Verwendung einer Kolonne im Sumpf, siehe die Ströme (5). Aus diesen hochsiedenden Anteilen kann das 1,3-Diol in üblicher Weise gewonnen werden, beispielsweise durch Destillation unter den oben genannten Bedingungen. Bei dieser Ausführungsform erfolgt die Bildung des Aldoladditionsproduktes ausgehend von einem einzigen Alkanal. Man erhält dann ein einziges, entsprechendes 1,3-Diol und zwar ausgehend von n-Butyraldehyd das EHD und ausgehend von i-Butyraldehyd das TMPD. Alternativ kann der aus der Destillation des Rohalkanalgemisches stammende hochsiedende Anteil, siehe Strom (6), der ein Gemisch an Aldoladditionsprodukten enthält, einer Hydrierung unterworfen werden, um ein 1,3-Diol zu erhalten. Vorzugsweise wird die Hydrierung jedoch zusammen mit der Hydrierung eines Alkanals vorgenommen, beispielsweise mit der Hydrierung von n-Butyraldehyd, siehe Strom (7).

Gemäss Figur 2 wird ein Teil des Alkanalrohgemisches, das durch Oxo-Synthese aus Propylen erhalten wurde, einer Destillation unterworfen, um die reinen Aldehyde zu gewinnen, siehe die Ströme (1), (2) und (3). Der verbleibende Teil des Rohgemisches wird einer Hydrierung unter den oben genannten Bedingungen unterworfen. Analog zu der Ausführungsform gemäss Figur 1 kommt es dabei zur Bildung des Aldoladditionsproduktes und zu dessen gleichzeitiger Hydrierung zu dem entsprechenden 1,3-Diol, siehe Strom (4). Das Produkt der Hydrierung wird dann einer Destillation unterworfen, beispielsweise einer Destillation in einer Kolonne oder mehreren Kolonnen wie oben beschrieben. Dabei werden n-Butanol und i-Butanol abdestilliert und aufgetrennt und entnommen, siehe Ströme (6) und (7). Das 1,3-Diol reichert sich bei der Destillation im hochsiedenden Anteil an, bei der Destillation in einer Kolonne im Sumpf. Zur Gewinnung des 1,3-Diols wird der hochsiedende Anteil seinerseits einer Destillation unterworfen, und zwar unter den oben genannten Bedingungen.

Alternativ kann der bei der Destillation des Aldehydgemisches anfallende hochsiedende Anteil (siehe Strom (9)), der ebenfalls das Aldoladditionsprodukt enthält, einer Hydrierung und Destillation zur Gewinnung des 1,3-Diols unterworfen werden. Vorzugsweise wird der hochsiedende Anteil gleichzeitig mit dem Aldehydgemisch hydriert, siehe Strom (10) und weiter wie oben beschrieben aufgearbeitet.

Die Menge der gebildeten 1,3-Diole beträgt etwa 40 bis 60 Gew.-% des hochsiedenden Anteils.

Bei der Ausführungsform gemäss Figur 2 wird, wie gesagt, ein Gemisch von i- und n-Butyraldehyd thermisch behandelt. Es bilden sich daher alle Kombinationen an Aldoladditionsprodukten, nämlich i/i, n/i, i/n, n/n. Durch Hydrierung erhält man dann ein Gemisch der entsprechenden 1,3-Diole. Dies wird nachfolgend anhand der Reaktionsgleichungen veranschaulicht.

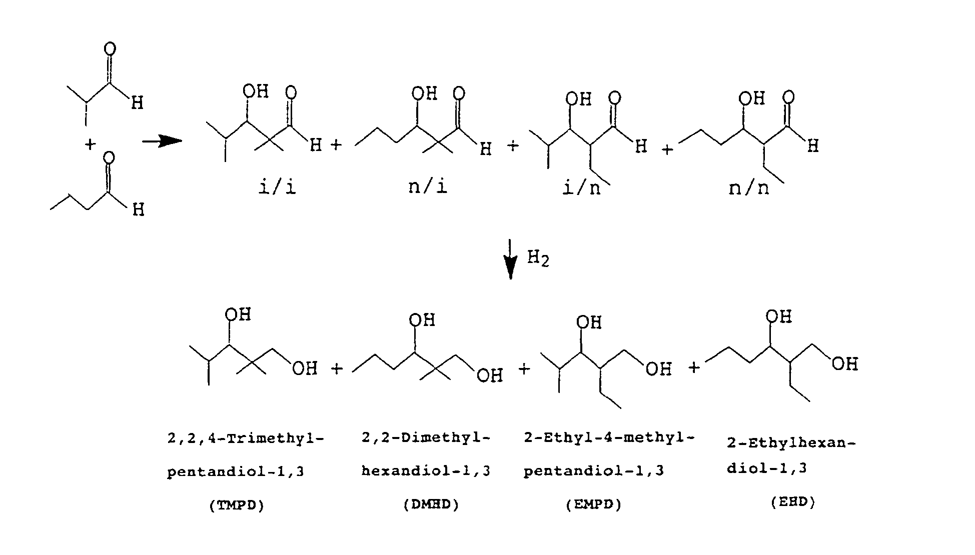

Trotz der hohen Anzahl an möglichen Produkten, die sich bei der thermischen Behandlung eines Alkanal-Gemisches bilden, kann das jeweils gewünschte 1,3-Diol in hoher Reinheit gewonnen werden. Im Falle der Verwendung eines Gemisches aus i- und n-Butyraldehyd kann EHD durch Destillation in hoher Reinheit erhalten werden. Als weitere Hauptkomponente fällt DMHD an, während TMPD und EMPD nur in geringen Mengen gebildet werden.

Das nachfolgende Beispiel erläutert die Erfindung ohne sie zu beschränken. Bei diesem Beispiel wird das erfindungsgemässe Verfahren anhand der Ausführungsform gemäss Figur 2 erläutert.

### Beispiel

Für die Destillation wurde der Rückstand aus der Herstellung von i- und n-Butanol eingesetzt, wie er nach der Verfahrensvariante gemäss Fig. 2 anfällt (Strom (8)). Die Kolonne (Nennweite 45 mm) wurde bei 20 mbar Kopfdruck mit 6 m CY-Packung (Fa. Sulzer) diskontinuierlich betrieben. Das Destillationsergebnis ist nachfolgend tabellarisch zusammengestellt.

| Fraktion | Temperatur | | Menge | Anteile (GC-FI%) | | | | |
|---|---|---|---|---|---|---|---|---|
| | [°C] | | [g] | Leichtsieder | 2-Ethylhexanol | DMHD | EHD | Rest |
| | | | 7430 Ausgangsmaterial | | 14 | 25 | 35 | |
| | Kopf | Sumpf | | | | | | |
| 1 | 62-71 | 128-135 | 788 | 99,3 | 0,14 | - | - | 0,56 |
| 2 | 85 | 141-143 | 834 | 2,94 | 95,77 | 0,06 | 0,94 | 0,29 |
| 3 | 123 | 150 | 452 | 1,1 | 42,14 | 40,0 | 0,9 | 15,86 |
| 4 | 125-128 | 152-154 | 1612 | 0,22 | 0,14 | 99,1 | 0,33 | 0,23 |
| 5 | 135 | 156 | 840 | 0,79 | - | 5,48 | 93,33 | 0,4 |
| 6 | 135-136 | 157-185 | 1744 | 0,56 | - | 0,12 | 99,22 | 0,1 |
| 7 | 155 | 197 | 483 | 28,88 | 0,03 | 2,41 | 20,1 | 48,28 |
| Sumpf | | | 677 | - | - | 0,01 | 0,03 | 99,96 |

## Patentansprüche

1. Verfahren zur Herstellung von 1,3-Diolen mit sechs oder mehr Kohlenstoffatomen, wobei das Verfahren die Schritte umfasst:
a) Bereitstellung mindestens eines Alkanals mit mindestens drei Kohlenstoffatomen;
b) thermische Behandlung des Alkanals in Abwesenheit eines basischen Katalysators;
c) Hydrierung des in Stufe b) gebildeten Aldoladditionsproduktes; und
d) Gewinnung des erhaltenen 1,3-Diols.

2. Verfahren nach Anspruch 1, wobei man das Alkanal thermisch behandelt, indem man es einer Destillation unterwirft.

3. Verfahren nach Anspruch 2, wobei man den hochsiedenden Anteil der Destillation der Hydrierung gemäss Stufe c) unterwirft.

4. Verfahren nach Anspruch 1, wobei man das Alkanal thermisch behandelt, indem man es einer Hydrierung unterwirft und das dabei gebildete 1,3-Diol gewinnt.

5. Verfahren nach einem der vorhergehenden Ansprüche, wobei das Alkanal 3 bis 10 Kohlenstoffatome aufweist.

6. Verfahren nach einem der vorhergehenden Ansprüche, wobei das in Stufe a) bereit gestellte Alkanal ein Gemisch verschiedener Isomere eines Alkanals ist.

7. Verfahren nach Anspruch 6, wobei das Gemisch das Reaktionsprodukt einer Oxo-Synthese ist.

8. Verfahren nach Anspruch 7, wobei das durch Oxo-Synthese erhaltene Gemisch ein Gemisch aus n-Butyraldehyd und i-Butyraldehyd ist.

9. Verfahren nach Auspruch 1 zur Herstellung von 2-Ethylhexandiol-1,3, wobei man:
i) ein Gemisch von n-Butyraldehyd und i-Butyraldehyd bereitstellt;
ii) das Gemisch einer thermischen Behandlung in Abwesenheit eines basischen Katalysators unterwirft, indem man es durch Destillation in n-Butyraldehyd und i-Butyraldehyd auftrennt;
iii) n-Butyraldehyd und i-Butyraldehyd zu n-Butanol bzw. i-Butanol, gegebenenfalls zusammen mit dem Destillationsrückstand von Stufe ii), hydriert und das Hydrierungsprodukt einer Destillation unterwirft; und
iv) 2-Ethylhexandiol-1,3 aus dem hochsiedenden Anteil der Destillation von Stufe iii) gewinnt.

10. Verfahren nach Anspruch 1 zur Herstellung von 2-Ethylhexandiol-1,3; 2,2-Dimethylhexandiol-1,3; 2,2,4-Trimethylpentandiol-1,3; und 2-Ethyl-4-methylpentandiol-1,3, wobei man:
i) ein Gemisch von n-Butyraldehyd und i-Butyraldehyd bereitstellt;
ii1) das Gemisch einer thermischen Behandlung gemäss Stufe b) und gleichzeitig einer Hydrierung gemäss Stufe c) unterwirft, indem man es hydriert; oder
ii2) einen Teil des Gemischs von Stufe i) einer thermischen Behandlung gemäß Stufe b) unterwirft, indem man es durch Destillation in n-Butyraldehyd und i-Butyraldehyd auftrennt und den Destillationsrückstand gegebenenfalls zusammen mit dem Gemisch von Stufe i) hydriert; und
iii) das Hydrierungsprodukt einer Destillation unterwirft und die 1,3-Diole aus dem hochsiedenden Anteil der Destillation gewinnt.

## Claims

1. A process for preparing 1,3-diols having six or more carbon atoms, which comprises the following steps:
a) provision of at least one alkanal having at least three carbon atoms;
b) thermal treatment of the alkanal in the absence of a basic catalyst;
c) hydrogenation of the aldol addition product formed in step b); and
d) isolation of the 1,3-diol obtained.

2. A process as claimed in claim 1, wherein the alkanal is thermally treated by subjecting it to a distillation.

3. A process as claimed in claim 2, wherein the high-boiling fraction from the distillation is subjected to the hydrogenation of step c).

4. A process as claimed in claim 1, wherein the alkanal is thermally treated by subjecting it to a hydrogenation and the 1,3-diol formed in this way is isolated.

5. A process as claimed in any of the preceding claims, wherein the alkanal has from 3 to 10 carbon atoms.

6. A process as claimed in any of the preceding claims, wherein the alkanal provided in step a) is a mixture of various isomers of an alkanal.

7. A process as claimed in claim 6, wherein the mixture is the reaction product from an oxo process.

8. A process as claimed in claim 7, wherein the mixture obtained from the oxo process is a mixture of n-butyraldehyde and i-butyraldehyde.

9. A process as claimed in claim 1 for preparing 2-ethylhexane-1,3-diol, in which:
i) a mixture of n-butyraldehyde and i-butyraldehyde is provided;
ii) the mixture is subjected to a thermal treatment in the absence of a basic catalyst by fractionally distilling it to give n-butyraldehyde and i-butyraldehyde;
iii) n-butyraldehyde and i-butyraldehyde are hydrogenated to n-butanol and i-butanol, respectively, if desired together with the distillation residue from step ii), and the hydrogenation product is subjected to a distillation; and
iv) 2-ethylhexane-1,3-diol is isolated from the high-boiling fraction of the distillation of step iii).

10. A process as claimed in claim 1 for preparing 2-ethylhexane-1,3-diol; 2,2-dimethylhexane-1,3-diol; 2,2,4-trimethylpentane-1,3-diol; and 2-ethyl-4-methylpentane-1,3-diol, wherein:
i) a mixture of n-butyraldehyde and i-butyraldehyde is provided;
ii1) the mixture is subjected to a thermal treatment as per step b) and is at the same time subjected to a hydrogenation as per step c), by hydrogenating it; or
ii2) part of the mixture from step i) is subjected to a thermal treatment as per step b) by fractionally distilling it to give n-butyraldehyde and i-butyraldehyde, and the distillation residue is, if desired together with the mixture from step i), hydrogenated; and
iii) the hydrogenation product is subjected to a distillation and the 1,3-diols are isolated from the high-boiling fraction from the distillation.

## Revendications

1. Procédé de préparation de 1,3-diols comportant 6 atomes de carbone ou davantage, dans lequel le procédé comporte les étapes :
a) une mise à disposition d'au moins un alcanal comportant au moins 3 atomes de carbone,
b) un traitement thermique de l'alcanal en l'absence d'un catalyseur basique,
c) une hydrogénation du produit d'addition aldol formé dans l'étape b), et
d) un isolement du 1,3-diol obtenu.

2. Procédé suivant la revendication 1, dans lequel on traite thermiquement l'alcanal en le soumettant à une distillation.

3. Procédé suivant la-revendication 2, dans lequel on soumet la fraction à point d'ébullition élevé de la distillation à l'hydrogénation selon l'étape c).

4. Procédé suivant la revendication 1, dans lequel on traite thermiquement l'alcanal en le soumettant à une hydrogénation et on isole le 1,3-diol ainsi formé.

5. Procédé suivant l'une des revendications précédentes, dans lequel l'alcanal comporte 3 à 10 atomes de carbone.

6. Procédé suivant l'une des revendications précédentes, dans lequel l'alcanal mis à disposition dans l'étape a) est un mélange de différents isomères d'un alcanal.

7. Procédé suivant la revendication 6, dans lequel le mélange est le produit réactionnel d'une synthèse oxo.

8. Procédé suivant la revendication 7, dans lequel le mélange obtenu par synthèse oxo est un mélange d'aldéhyde n-butyrique et d'aldéhyde i-butyrique.

9. Procédé de préparation de 2-éthylhexane-1,3-diol suivant la revendication 1, dans lequel
i) on met à disposition un mélange d'aldéhyde n-butyrique et d'aldéhyde i-butyrique,
ii) on soumet le mélange à un traitement thermique en l'absence d'un catalyseur basique, en le séparant par distillation en aldéhyde n-butyrique et aldéhyde i-butyrique,
iii) on hydrogène de l'aldéhyde n-butyrique et de l'aldéhyde i-butyrique en n-butanol ou respectivement i-butanol, éventuellement conjointement au résidu de distillation de l'étape ii), et on soumet le produit d'hydrogénation à une distillation, et
iv) on isole du 2-éthylhexanediol-1,3 à partir de la fraction à point d'ébullition élevé de la distillation de l'étape iii).

10. Procédé de préparation de 2-éthylhexane-1,3-diol, de 2,2-diméthylhexane-1,3-diol, de 2,2,4-triméthylpentane-1,3-diol et de 2-éthyl-4-méthylpentane-1,3-diol suivant la revendication 1, dans lequel
i) on met à disposition un mélange d'aldéhyde n-butyrique et d'aldéhyde i-butyrique,
ii1) on soumet le mélange à un traitement thermique selon l'étape b) et simultanément à une hydrogénation selon l'étape c), en l'hydrogénant, ou
ii2) on soumet une partie du mélange de l'étape i) à un traitement thermique selon l'étape b), en la séparant par distillation en aldéhyde n-butyrique et aldéhyde i-butyrique, et on hydrogène le résidu de distillation éventuellement conjointement au mélange de l'étape i), et
iii) on soumet le produit d'hydrogénation à une distillation et on isole les 1,3-diols à partir de la fraction à point d'ébullition élevé de la distillation.
